# EUROPEAN PATENT SPECIFICATION

(11) **EP 0 535 007 B1**
(45) Date of publication and mention of the grant of the patent: **09.08.1995**
(21) Application number: 91909824.4
(22) Date of filing: 21.05.1991
(51) Int. Cl.: C07K 16/10, G01N 33/577

(54) **A MONOCLONAL ANTIBODY AGAINST THE GAG PROTEIN PRECURSOR p55 OF HUMAN IMMUNODEFICIENCY VIRUS**
MONOKLONALE ANTIKÖRPER GEGEN GAG-PROTEIN-VORLÄUFER-P55 VON HIV-VIRUS
ANTICORPS MONOCLONAL ANTAGONISTE DE LA PROTEINE PRECURSEUR p55 GAG DU VIRUS D'IMMUNODEFICIENCE HUMAINE

(30) Priority: 01.06.1990 EP 90110461; 17.07.1990 EP 90113658; 29.11.1990 EP 90122813
(43) Date of publication of application: 07.04.1993
(73) Proprietor: GRUPPO LEPETIT S.p.A., I-20020 Lainate (MI) (IT)
(72) Inventor: NOLLI, Maria, Luisa, Cesira, I-21053 Castellanza (IT); SARUBBI, Edoardo, Giacomo, I-20146 Milano (IT)
(74) Representative: Macchetta, Francesco
(86) International application number: EP9100947
(87) International publication number: WO9119000

(56) References cited:
- WO-A-89/04376
- WO-A-89/04488

## Description

The human immunodeficiency virus (HIV) is the causative agent of the acquired immunodeficiency syndrome (AIDS). HIV is a member of the lentivirus sub-family of retroviruses and infects human CD4+T-lymphocytes, macrophages, and perhaps other cell types (see A.S. Fauci: "The human immunodeficiency virus: Infectivity and mechanisms of pathogenesis", Science 239:617-622, 1988. D.D. Ho, R.J. Pomerantzk and J.C. Kaplan: "Pathogenesis of infection with human immunodeficiency virus", N.Engl.J.Med. 317:278-286, 1987). The genome of the virus, like other retroviruses, contains gag, pol and env genes, which code for the viral core proteins, viral enzymes including the reverse transcriptase and the viral envelope protein, respectively. The virus also contains at least five additional genes, three of which have regulatory functions and the expression of these genes almost certainly has an impact on the pathogenic mechanism exerted by the virus.

Several approaches to discover drugs for anti-HIV therapy have been followed in the last few years (see J.S. Oxford, A.R.M. Coates, D.Y.Sia, K.Brown and S. Asad: "Potential target sites for antiviral inhibitors of human immunodeficiency virus (HIV)", J.Antimicrob. Chemother. 23 Suppl. A, 9-27, 1989).

Potential targets for anti-HIV drugs are the essential viral functions such as the entry of the virus into the cell, enzymatic activities and the cellular activation and proliferation. These functions are regulated by the expression of viral genes. In the case of the gag, pol and env genes, they encode for precursor polypeptides that are post-translationally modified by proteolytic processing and other modifications. The enzymatic activities derived from the pol gene polypeptide precursor including protease, reverse transcriptase and endonuclease are considered interesting targets for inhibitors. The protease cleaves the precursor product of the gag gene, the p55 protein, into 4 structural proteins of the viral core, among which there is p24, the major internal core protein and p17. These two proteins derive from the splitting of contiguous amino acid sequences of the p55 precursor. The same protease also cleaves the gag-pol precursor to yield the protease itself (autolytic activation), the reverse transcriptase and the endonuclease (see W.C. Robey, B. Safai, S.Oroszlan, L.O. Arthur, M.A. Gonda, R.C. Fallo and P.J. Fischinger: "Characterization of envelope and core structural gene products of HTLV-III with sera from AIDS patients", Science 228:593-595, 1985. I. Katoh, T.Yasunaga, Y.Ikawa and Y. Yoshinaka: "Inhibition of retroviral protease activity by an aspartyl proteinase inhibitor", Nature 329:654-656, 1987. C. Debouck, J.G. Gorniak, J.E. Strickler, T.D. Meek, B.W. Metcalf and M. Rosenberg: "Human immunodeficiency virus protease expressed in Escherichia coli exhibits autoprocessing and specific maturation of the gag precursor", Proc. Natl. Acad. Sci. 84:8903-8906, 1987).

Detection of the presence or exposure to HIV-1 in patients has been one of the major targets of scientific research and commercial application during the most recent years.

Assays for HIV-1 detection are generally based on immunological reactions and inactivated whole virus is employed as the antigen reagent to generate appropriate antibodies. Antibodies against HIV-1 proteins essential for viral replication are also described in the recent patent and scientific literature, as well as their use in both the early detection of HIV-1 antigens and in the monitoring of the antiviral therapy in patients infected with HIV-1. In particular, a mouse monoclonal antibody to HIV-1 gp41 is described in EP-A Publication No. 335134. Mouse monoclonal antibodies with specific reactivity toward HIV-1 p24 are disclosed in EP-A Publication No. 345461. Monoclonal and polyclonal antibodies specific toward gp48 are described in International Patent Appln. Publ. No. WO 90/00617.

Use of monoclonal antibodies reactive with p24 and p17 to detect presence of p55, p24 and p17 through western blot analysis of cell lysates of T-lymphocytes chronically infected with HIV-1 is disclosed by T.D. Meek et al: "Inhibition of HIV-1 protease in infected T-lymphocytes by synthetic peptide analogues", Nature, 343, 90-92 (1990).
Recombinant fusion viral core proteins and the production of the respective antibodies are disclosed in EP-A Publication No. 305777.

This invention provides a monoclonal antibody against the precursor product of the gag genes, that is the protein p55, or a portion thereof which contains the p55 cleavage site by the HIV-1 protease which generates the p24 and p17 polypeptides ("p24/p17 HIV-1 protease cleavage site"). The monoclonal antibody of this invention is useful for detecting the presence of the gag precursor of both p24 and the other smaller polypeptides in biological fluids and/or tissues containing it for the purpose of monitoring the protease activity as marker of the viral activity, for instance in persons who have been exposed to HIV-1 and have not yet developed AIDS or patients who are undergoing therapeutical treatment. The term "biological fluids and/or tissues" includes besides mammalian whole blood, plasma,lymphocytes and cell cultures also cellular or bacterial extracts derived from the expression of gag gene in eukaryotic or prokaryotic host as well as any mixture or solution which contain natural or synthetic polypeptides containing the same aminoacid sequence of p55 or a fragment thereof which contains the p55 cleavage site by the HIV-1 protease which generates the p24 and p17 polypeptides.

Therefore, one of the main uses of the antibody of this invention is in enzymatic immunoassays of serum and other body fluids and/or tissues of people exposed to HIV-1.

A further use of the antibody is in the isolation and/or purification of natural or synthetic peptides containing the p55 cleavage site by the HIV-1 protease which generates the p24 and p17 polypeptides. This permits obtainment of the precursor protein in a desirable amount for investigations of the viral replication mechanism and for producing and isolating substances competing with said precursor towards the HIV-1 protease and representing potential prophylactic and/or therapeutical agents.
According to such uses the antibody may be bound to an inert support to form an affinity matrix according to the method known in the art (see, for instance, J.W. Goding: Monoclonal Antibodies Principles and Practice, Academic Press, New York, 1983, 188-207).

Some specific examples for the practical uses of the antibody of this invention may also be drawn by analogy from EP-A Publication No. 345461 and EP-A publication No. 335134.
As a peculiar characteristic of the antibody of this invention is the specific reactivity with an epitope of the p55 protein which comprises the cleavage site of the HIV-1 protease which generates the p24 and p17 polypeptides, this antibody is particularly suitable for devising immunoassay tests for screening both known and unknown substances for HIV-1 protease inhibitory activity in fluids, tissues and cultured cells. In fact, the tests presently used for detecting such activity usually rely on polyclonal or monoclonal antibodies mainly directed against the viral polypeptides resulting from the cleavage of the gag precursor. Said antibodies cannot discriminate between gag precursor and cleaved polypeptide product (either p17 or p24) and therefore are commonly used as primary antibodies in Western blot techniques (see T.D. Meek et al, Nature, 343, 90-92, 1990 and McQuade et al: "A synthetic HIV-1 protease inhibitor with antiviral activity arrests HIV-like particle maturation", Science, 247, 454-456, 1990).
Briefly, according to known methods, the proteins contained in fluids, tissues or cell lysates are separated by polyacrylamide gel electrophoresis (PAGE) and then blotted on nitrocellulose filters. The HIV proteins which are recognized by the primary antibody are subsequently visualized by incubation with a conveniently labelled secondary antibody. Consequently, it is possible to compare the ratio between precursor and product in untreated samples with the same ratio in samples treated with an inhibitor of the protease reaction and therefore obtain information about the efficacy of such inhibitor inside the treated samples. The availability of an antibody which recognizes only the precursor and not any protease cleaved product makes possible to avoid the PAGE for the protein separation step. An example is the use of a sandwich ELISA-type assay. Accordingly, it is possible to immobilize such antibody on a solid support such as microtiter wells and to add to these wells the fluid, tissue or cell lysates from HIV-infected cells which contain the protease substrate. Consequently, only the gag precursor (a fragment thereof or a synthetic polypeptide containing the same aminoacid sequence of the p24/p17 cleavage site region: "precursor-like polypeptides") is bound while all the other proteins, of viral and cellular (or synthetic) origin, can easily be washed away. The amount of gag precursor or precursor-like polypeptide can be subsequently determined by incubating with a different antibody (either -mono- or polyclonal) which recognizes a different epitope of the precursor (either on the p24 or the p17 portion of it) and which has been previously labelled with a marker enzyme (for example, horse radish peroxidase or alkaline phosphatase and others).
Examples of different possibilities for carrying out such an assay, together with a description of reaction conditions can be found in the Chapter: "Practice and theory of enzyme immunoassays" by P. Tijssen in the volume: "Laboratory techniques in biochemistry and molecular biology", edited by R.H. Burden and P. H. van Knippenberg, Elsevier, 1985.
The selective antibody of this invention may be associated with other reagents and means necessary to perform the analytical assay in a ready-to-use kit.

A further use of this antibody is for the screening of potential inhibitors of HIV-1 protease in a test which utilizes as protease specific substrate an HIV-gag fusion protein containing a p55 fragment, which in turn comprises a portion of both p24 and p17 and includes the p24/p17 HIV-1 protease cleavage site, bound to a marker enzyme, e.g. β-galactosidase. This fusion protein may be prepared , for instance, by isolating the DNA fragment which codes such protein fragment from the viral genome of the HIV strain LAV and inserting such DNA fragment into the structural gene of E. coli β-galactosidase contained in the vector plasmid pVR292. Such insertion being in the same reading frame of the β-galactosidase coding sequence allows the expression of the gag protein fragment in E. Coli as a protein fused with the β-galactosidase enzyme.

An example of such immunoassay is briefly outlined in the following procedure:
1) incubation of the potential inhibitor with a solution containing the HIV-1 protease and the HIV-gag fusion protein
2) incubation of the above reaction mixture with the immobilized monoclonal antibody of this invention. This allows the selective binding of uncleaved HIV-gag fusion protein to the antibody;
3) removal of the test solution to separate the products resulting from the HIV-1 activity (cleaved HIV-gag fusion protein) from the uncleaved HIV-gag fusion protein which remains bound to the immobilized antibody;
4) addition of a reactant of the marker enzyme portion of the fusion protein, e.g. a β-galactosidase-specific chromogenic substrate such as p-nitrophenyl-galactopyranoside (PNPG), and determination of the enzymatic activity. This last step allows the quantitation of the HIV-gag fusion protein which has not been cleaved by the HIV-1 protease and hence affords the measurement of the HIV-1 protease activity by comparison with a blank sample which does not contain the potential inhibitor.

This assay is very efficient and since it can be performed in microtiter wells, it is particularly suitable for the rapid screening of a large number of samples. For such purpose automation is also feasible. The test samples could be of any origin. They can be compounds of known or unknown structure, derived from chemical synthesis or natural products. Also, they can be purified substances or complex mixtures, i.e. cellular extracts, microbial fermentation broths, solutions containing synthetic products or extracts of biological materials, including vegetables.

This assay is also relatively unaffected by the presence of low concentrations (5-10%) of organic solvents like methanol, acetonitrile or DMSO, a feature particularly useful when the assay is used to monitor the purification of an inhibitory activity.

This invention includes also a method for preparing the selective monoclonal antibody and the analytical assays or purification procedures which employ it. The preferred method of production of the antibody of this invention is particularly useful for producing commercial amounts of this latter, since it does not involve direct use of virus or essential viral protein or polypeptides as the antigen, which is potentially hazardous but, rather, the use of the "inactive" p55 precursor or, preferably, a genetically engineered fusion protein containing portions of p24 and p17 polypeptides which comprise the cleavage site of HIV-1 protease which generates the two polypeptides. The monoclonal antibody of this invention can be produced by antibody producing cell lines, which usually are hybridoma cell lines. The hybridomas are formed by fusion of antibody producing cells and a stabilized cell line which imparts long term tissue culture stability to the hybrid cells. The antibody producing cells may be spleen cells from animal immunized against the protein p55 or a portion thereof which contains the p24/p17 HIV-I protease cleavage site. The animals immunized are usually small mamalians, preferably rodents, such as rats or mice. The second fusion partner, the stabilized cell line, may be a lymphoblastoid cell line or a myeloma cell line, preferably, syngenic with the first fusion partner. Several cell lines of this type are known and available to the public from public collections such as the American Type Culture Collection (ATCC), Rockville, Maryland (USA). An example of said cells is the HAT (hypoxantine, aminopterin, thymidine) sensitive non-producer mouse myeloma cell line Sp 2/0-Ag14.
The fusion technique is usually following the standard procedure described by C. Milstein and G. Köhler, Nature, 256, 495-497 (1975) and reviewed in "Monoclonal Hybridoma Antibodies: Techniques and Applications", editor J.G.R. Hurrell C.R.C. Press Inc. (1982).
Although the antigen useful for the immunization of the animal may be composed of inactivated whole HIV-1 virus, or partially purified natural p55 protein, cloned proteins containing the p55 sequence or a fragment thereof which comprises the p24/p17 cleavage site are preferably employed. Recombinant proteins of this type have the advantage to be obtained in amounts sufficient to carry out the process of this invention without hazardous handling of HIV-1 infected host materials, as it would require the utilization of the natural p55 protein. The patent and scientific literature report methods of expression of HIV-1 gag fusion proteins which can provide fusion proteins useful as antigens for immunizing the animals to obtain the epitope specific monoclonal antibody of this invention. See for instance EP-A Publ. No. 305777, EP-A Publ. No. 345461 and C. Guenet et al, European Journal of Pharmacology -Molecular Pharmacology Section, 172, 443-451 (1988).
A particularly suitable antigen for producing the antibody of this invention is a HIV-gag fusion protein containing the p55 fragment, which in turn comprises a portion of both p24 and p17 and includes the p24/p17 HIV-1 protease cleavage site, fused with a suitable marker enzyme, e.g. β-galactosidase. A typical example of said fusion protein is the HIV-gag fusion protein which contains 110 aminoacid residues fused with β-galactosidase (gal-gag 110)described in Example 1. According to a preferred embodiment of this invention, the antibody showing specific reactivity with an epitope of the p55 protein which comprises the p24/p17 cleavage site of the HIV-1 protease can be produced by: (i) immunizing mice with the above mentioned fusion protein; (ii) recovering spleen cells from immunized mice and fusing them with HAT sensitive non-producer mouse myeloma Sp 2/0-Ag 14 cells; (iii) testing the obtained hybrids by an appropriate immunoassay for the production of monoclonal antibodies against the p55 portion of the fusion protein; (iv) sub-cloning the cells positive to the previous assay; (v) analyzing the subcloned cells for the epitope specificity and selecting for larger cultivation those hybridomas which secrete an antibody directed specifically to the p24/p17 HIV-1 protease cleavage site of the fusion protein; (vi) isolating and purifying the specific antibody according to the methods usually employed in the art.

In general, the immunization step is carried out by using a purified preparation of the fusion protein containing both the p24 and p17 portions. The animals are given 3 to 5 injections spaced 12 to 17 days after the previous one. A satisfactory titre of antibodies is usually obtained sixty days after the start of the immunization. The mice are usually given a final booster 2 to 5 days before sacrification. The plasma and other lymphoid cells removed from the spleen are fused with the mouse myeloma cell, usually in the presence of a fusion promoter such as polyethylene glycol 2500. Following the fusion, the myeloma cells are eliminated by addition of HAT medium while the non-hybridized spleen cells die spontaneously in a few days.
The antibody producing hybridomas are determined by testing the supernatants by an appropriate anti-HIV gag protein enzyme-linked immunoassay (ELISA) whereby the reactivity of the antibodies secreted by the hybridoma toward the fused HIV-gag/β-galactosidase protein is compared with that toward β-galactosidase alone. The hybridomas furnishing supernatants which are positive to the fusion protein and negative to the β-galactosidase are sub-cloned and their supernatants are analyzed for epitope specificity by testing the reactivity toward the fusion protein before and after cleavage by the HIV-1 protease by immunoblotting. The clones which yield supernatants demonstrating good affinity for the fusion protein before cleavage and which do not bind to the same protein when cleaved by the protease are selected for further subcloning and mass culture.
As a representative example of the method of selection of the selective antibody producing hybridomas, in two fusion experiments, 880 clones were screened. Twenty one clones were found positive on both gal-gag 110 fusion protein and β-galactosidase, 8 were positive only on β-galactosidase and 7 only on gal-gag 110 fusion protein. The 7 clones recognising the gal-gag 110 fusion protein were sub-cloned and selected for further characterization for epitope specificity. Among these 7 clones, 4 clones were reconfirmed for positivity on gal-gag 110 fusion protein before cleavage and only one, named 1G12, which demonstrated a good affinity for the fusion protein, did not show any binding after cleavage of the fusion protein. The clone producing the specific antibody was then selected for further subcloning and mass cultivation which may be carried out according to common techniques. For instance, by amplification in mice or by cell culture in a hollow fiber bioreactor. The isolation and purification of the 1G12 antibody can be carried out by applying a solution containing said antibody to an affinity chromatography column, for instance, a Staphylococcus aureus Protein-A-Sepharose column previously equilibrated with a binding buffer pH 8.9, washing the unbound proteins by addition of binding buffer and then eluting the bound fraction with a specific elution buffer at pH 6. The recovered antibody is then dyalyzed against PBS (phosphate buffer saline) tested by SDS-PAGE (sodium dodecylsulfate-polyacrylamide gel electrophoresis) in reducing conditions for homogeneity and stored at -20°C (minus twenty °C).
The purified monoclonal antibody 1G12 showing specific reactivity toward an epitope of the p55 HIV gag protein precursor which contains the p24/p17 protease cleavage site is further characterized by class specificity by an ELISA assay and is found to belong to the IgG₁ class and to possess light chains of the k type.
The SDS-PAGE analysis in reducing and non reducing conditions indicates an approximate molecular weight of the order of 150,000 Dalton.
The approximate value of the affinity constant of the 1G12 antibody toward the gal-gag 110 fusion protein containing portions of p24 and p17 protein with the p24/p17 protease cleavage site and covalently bound to β-galactosidase is 5x10⁹ M⁻¹.

### EXAMPLES

The following examples illustrate a method utilized to embody this invention without limiting its scope. The following materials, methods, cell lines and media were employed besides those specifically described in the Examples and Figures. All chemicals used were of analytical grade.

### MATERIALS

The following special materials were used: Protein-A Sepharose (Pharmacia, Uppsala, Sweden); sheep anti-mouse Ig, horseradish peroxidase linked F(ab')₂ fragment (Amersham, international Little Chalfront, Buckingamshire, England), goat anti-mouse Igs (Janssen Biochimica, Beerse, Belgium), humane endothelial cell supernatants (HECS) from Janssen.

### CELL LINES AND MEDIA

The HAT sensitive non-producer mouse myeloma cell line Sp2/0 - Ag14 (M.C.Shulman, C.D.Wilde and G. Köhler: "A better cell line for making hybridomas secreting specific antibodies"; Nature 276, 269-270, 1978) used for the fusion experiments was grown in Dulbecco's modified Eagle medium (DMEM) supplemented with 10% foetal calf serum (FCS, Flow laboratories), 1mM glutamine, 10 U/ml penicillin, 0.01 mg/ml streptomycin. The hybridoma lines were maintained in DMEM with 5% FCS, 5% HECS, HAT (0.1 mM hypoxanthine/0.4 »M aminopterin/16 »M thymidine), 1mM glutamine, 10 U/ml penicillin, 0.01 mg/ml streptomycin.

### Example 1: Preparation and characterization of the gal-gag 110 fusion protein containing the p24/p17 HIV-1 cleavage site

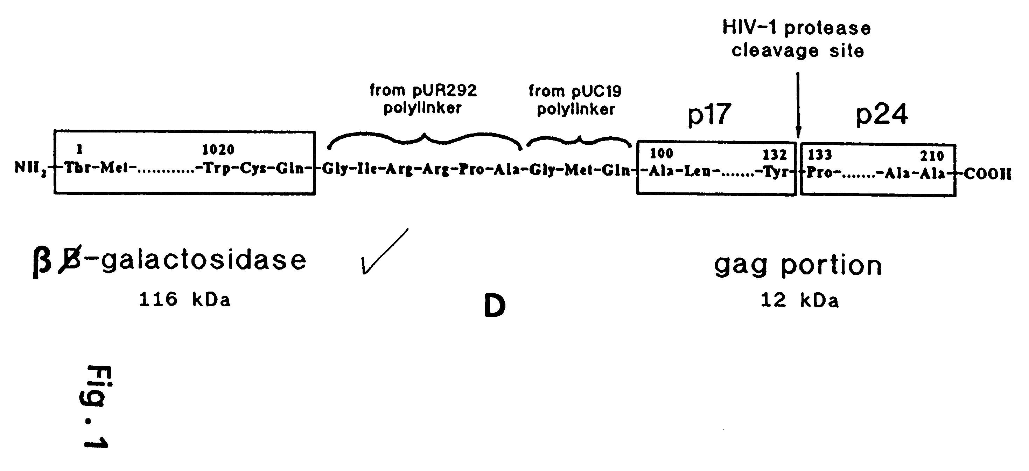

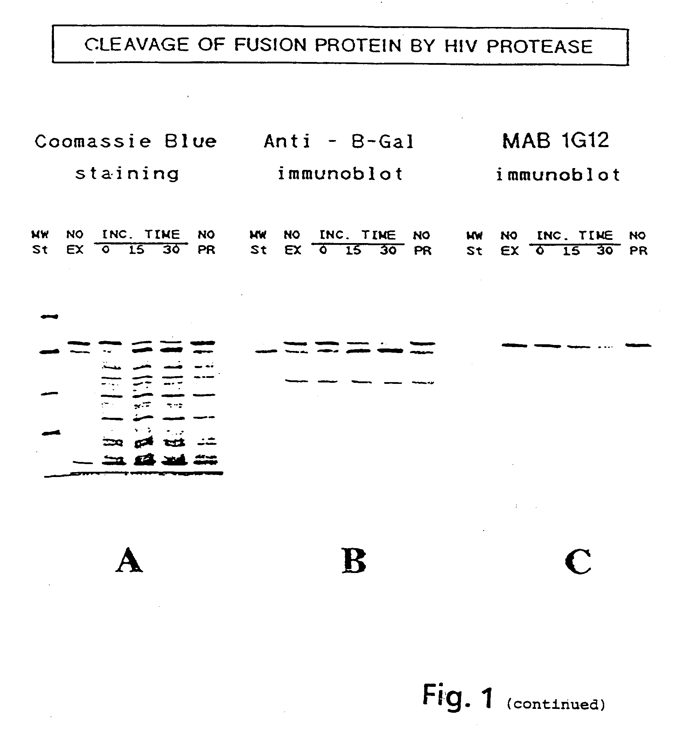

The Hind III-Hind III fragment from bp 631 to 1258 of HIV-gag DNA (A. Wain-Hobson, P. Sonigo, O. Danos, S. Cole and M. Alizon: Cell 40, 9-17, 1985) was subcloned into the unique Hind-III site of pUC19 (GIBCO BRL, International Division, Gaithersburg, MD). From the resulting construct the PstI-PstI fragment containing 8 bp from the pUC19 polylinker (PstI-Hind III) fused to the Hind III-PstI fragment from bp 631 to 961 of HIV-1 DNA, was isolated and inserted into the unique PstI site of pUR292 (U. Ruther and B. Muller-Hill: EMBO J., 2, 1791-1794, 1983). This gave plasmid pGA22 in which the gag fragment is fused in frame to the end of the lacZ gene so that a fusion protein is encoded. The structure of this "gal-gag 110" fusion protein is depicted in Fig. 1D.
The plasmid pGA22 was used to transform E. coli strain JM109.
E. coli JM109 (pGA22) was grown at 37°C in LB medium with 50 »g/ml ampicillin. At OD₆₀₀=0.5 IPTG was added to 1 mM final. After 2 hours bacteria were harvested and resuspended in 1/50 volume of 50 mM tris-HCl, pH 8, 50 mM NaCl, 1 mM EDTA (Buffer A), containing 0.1 mM PMSF, 1 mM benzamidine-HCl, 0.1 M arginine and 1 mg/ml lysozyme. After 30 minutes at 4°C cells were disrupted by sonication and centrifuged for 10 minutes at 25,000 g. The pellet was washed twice by resuspension in Buffer A containing 70 mM 2-mercaptoethanol (2-ME) and 0.05% Triton-X100 and centrifuged at 25,000 g. The pellet was finally dissolved in 0.1 M Tris-HCl, pH 7.8, 0.1 M NaCl, 1mM MgCl₂, 0.5 mM EDTA, 70 mM 2-ME (Buffer B), containing 8 M urea. After centrifugation for 20 minutes at 50,000 g, the supernatant was first diluted with Buffer B to 5 M urea, then extensively dialyzed against Buffer B without 2-ME. Affinity chromatography on TPEG-Sepharose was performed essentially as described (A. Ullmann: Gene, 29, 27-31, 1984).
Recovery of gal-gag 110 in each purification step was checked by SDS-PAGE with the PHAST-GEL system (Pharmacia).

The purified fusion protein was examined by SDS-PAGE where it gave a major band of about 130,000 Da (see Fig. 1A, lane NO EX). This corresponds to 116,000 Da, the molecular weight of β-galactosidase, plus about 14,000 Da, the expected molecular weight of the gag portion fused to the enzyme. Such protein is indeed cleaved by the HIV protease (as shown in Fig. 1A, lanes 0, 15 and 30) to yield a band at about 120,000 Da.
The HIV protease employed was a recombinant HIV-1 protease obtained by expression of a synthetic gene in E. coli according to C. Guenet et al., European Journal of Pharmacology - Molecular Pharmacology Section, 172, 443-451 (1989).
An immuno-blot analysis of the purified fusion protein before and after cleavage by the HIV protease (Fig 1B) shows that such protein is recognized by a monoclonal antibody specific for E. coli β-galactosidase (Boehringer Mannheim, W. Germany) both before and after cleavage.
The resulting protein contains the Ala¹⁰⁰ to Ala²¹⁰ fragment from HIV-1 gag polypeptide (p55) fused to the carboxy-terminus of E. coli β-galactosidase (Fig. 1D). The gag portion of this "gal-gag 110" fusion protein comprises the p17/p24 HIV-1 protease cleavage site Tyr¹³²-Pro¹³³ (A. Wain-Hobson, P. Sonigo, O. Danos, S. Cole and M. Alizon: Cell 40, 9-17, 1985), while the β-galactosidase portion provides an easily measurable enzymatic activity.

### Example 2: Production of the monoclonal antibodies against gal-gag 110 fusion protein

Female Balb/c mice were immunized by injecting into the peritoneum of the animals 0.050 mg of the fusion protein of Example 1 in complete Freund's adjuvant (diluted 1:1) at day -60. The same quantity in incomplete Freund's adjuvant was injected in the peritoneum of the animals at day -42, -30,-18. At day -3 a final booster of 0.05 mg was given intravenously to the animals. To obtain the monoclonal antibody, at day 0 spleen cells (5 x 10⁷) of the immunized mice were fused to 1 x 10⁷ HAT sensitive non-producer mouse myeloma Sp2/0-Ag 14 cells. The fusion was carried out with polyethylene glycol 1500 and then cells were plated onto five different 96-well microtiter plates (Costar, One Alewife Center, Cambridge, MA, USA) in DMEM supplemented with 5% FCS, 5% HECS, HAT, 1mM glutamine, 10 U/ml penicillin, 0.01 mg/ml streptomycin. Supernatants of the growing hybrids were tested every 2-3 days by an enzyme-linked immunoadsorbent assay (ELISA) for production of monoclonal antibodies against the portion of the gal-gag 110 protein (See example 3).

### Example 3: Anti-gal-gag 110 protein monoclonal antibody enzyme-linked immunoadsorbent assay

96-well microtiter plates (Falcon-Beckton Dickinson) were coated at alternate strips with 0.05 ml/well (0.000125 mg/well) of a) gal-gag 110 fusion protein in PBS (phosphate buffer saline), 0.1 M sodium chloride pH 7.3 or of b) β-galactosidase in the same buffer. After an overnight incubation at 4° C plates were washed with PBS and incubated with 1% BSA (bovine serum albumin) pH 7.3 in PBS for one hour and half at room temperature. Then 0.05 ml/well of supernatants of hybridomas were added and left to incubate for two hours at room temperature. After washing with PBS, 0.005% Tween 20, 0.05 ml/well of an anti-mouse Igs peroxidase conjugated F(ab')₂ fragment was added. After one hour and half plates were washed and "incubated with 0.15 ml/well of a solution of 1 mg/ml of ortho-phenylenediammine in 0.1 M citrate buffer, 1.75 mM H₂0₂, pH₅. The coloured reaction was stopped with H₂SO₄ 4.5 M. The results were read using Titertek Multiskan spectrophotometer (Flow Laboratories, Irvine, Scotland, UK) at 492 nm.
With this procedure were screened 880 clones obtained in two fusion experiments carried out as described in Example 2. Of these clones, 21 were found positive on both gal-gag 110 fusion protein and β-galactosidase, 8 were positive only on β-galactosidase and 7 only on gal-gag 110 fusion protein. The seven clones recognizing the gal-gag 110 fusion protein were selected for further subcloning by limiting dilution technique and confirmation for positivity toward gal-gag 110 fusion protein. Only four clones were confirmed and successively tested for epitope specificity according to Example 4.

### Example 4: Selection of the hybridoma secreting monoclonal antibody 1G12 through epitope characterization

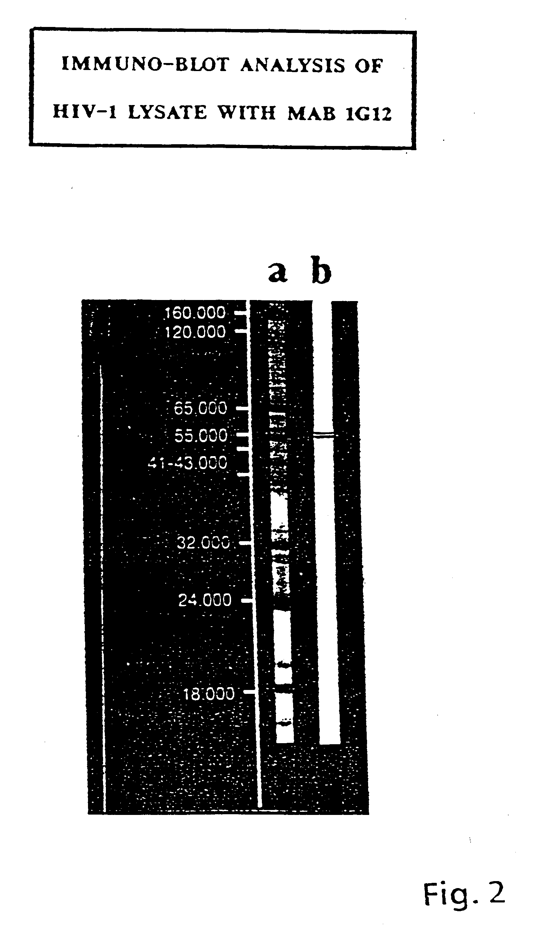

A Western blot analysis of the gel electrophoresis of the gal-gag 110 fusion protein before and after incubation with HIV-1 protease was carried out by using the supernatants of the four clones confirmed positive to gal-gag 110 fusion protein (see Example 3) as primary antibody according to the conditions given in the Figure 1C.
HIV protein detection kit (Novapath Immunoblot Strips, BIORAD, Richmond, CA, USA) containing HIV-1 total viral proteins blotted onto different nitrocellulose strips was used following the protocol described in Figure 2.
Only one of the four clones showing affinity for the gal-gag 110 fusion protein showed no binding with the gal-gag 110 protein after incubation with the protease (see Figure 1C). The clone secreting this antibody was selected for further larger scale production of the monoclonal antibody and for its isolation and purification. The antibody was named 1G12.

### Example 5: Scale production and purification of antibody 1G12

### a) Scale production:

Two methods were used to produce large quantities of monoclonal antibody 1G12:
1) the mouse system consisting of the injection of 1-2 x 10⁶ hybridoma cells/mouse previously sensitized with pristane. From 10 to 15 days after the injection, ascitic fluid, containing the antibody in the range of 1-10 mg/ml of ascitic fluid was recovered (1-10 ml) from the peritoneum of mice.
2) The cell culture system based on an hollow fiber bioreactor (Vitafiber VLS - Amicon-Amicon Division, W.R. & Co. Danvers, MA, USA).
   This system combines a Flo-Path bioreactor (a cartridge of 3700 cm³) in which cells grow with feed and recirculation pumps, temperature controlled incubator, pH controller and air supply.
   5 x 10⁸ hybridoma cells were inoculated in the extracapillary space of the cartridge with the medium feed (DMEM + 10% FCS + 1mM glutamine, 10 U /ml penicillin, 0.01 mg/ml streptomycin) passing through the lumen of the fibers (cut off 30,000 Da) and feeding cells growing in the extracapillary space. The monoclonal antibody (150,000 Da) was concentrated into the extracapillary space and was harvested daily. Using this system several grams of monoclonal antibody 1G12 were produced.

### b) Recovery

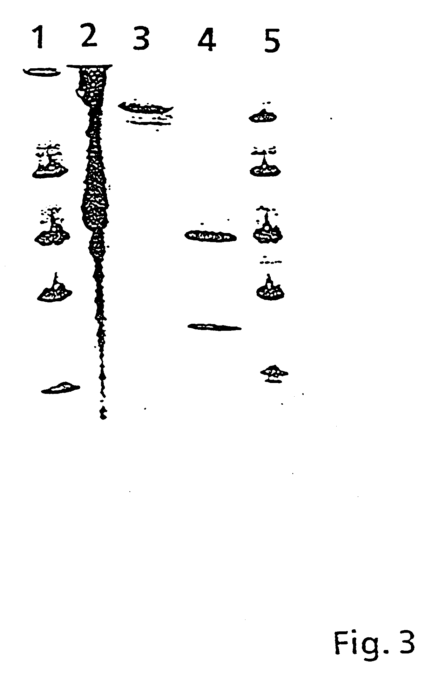

Monoclonal antibody 1G12 was purified from fluid according to the following protocol: 10 ml of the fluid were mixed with 20 ml of acetate buffer 0.06 M, pH 4 and with 0.5 ml of octanoic acid (caprylic acid, Merck AG, Darmstadt, West Germany), then vigorously shaked for 30 minutes at room temperature. The solution was then centrifuged at 20,000 rpm for 30 minutes, at 15°C, in a Beckman SW 28 rotor. The supernatant was collected and dialyzed overnight against phosphate buffer saline, 0.02 M sodium chloride pH 7.3. The dialyzed solution (1ml) was applied on a Staph. aureus Protein A-Sepharose CL4B (Pharmacia LKB Biotechnology, Uppsala, Sweden) column, previously equilibrated with a binding buffer, 1.5 M glycine, 3 M sodium chloride pH 8.9. The unbound proteins are washed out with 5 column volumes of binding buffer, while the bound fraction was eluted with the specific elution buffer (0.1 M citric acid pH 6). The column was cleaned with a regeneration buffer (0.1 M citric acid pH 3). Monoclonal antibody 1G12 was dialyzed against PBS, tested by SDS-PAGE in reducing and non reducing conditions for homogeneity (see Figure 3) and stored at -20°C.

### Example 6: Characterization of the monoclonal antibody 1G12

The monoclonal antibody 1G12 was tested for class specificity by enzyme-linked immunoadsorbent assay using the BIORAD kit (Mouse-Typer Sub-isotyping Panel, including: Rabbit-anti-mouse subclass specific to IgG₁, IgG₂ₐ, IgG_{2b}, IgG₃, IgM, IgA, k chain, λ chain). The results of this test show that monoclonal antibody 1G12 belongs to IgG₁ class and that its light chains are of type k.
Molecular weight: The molecular weight of the monoclonal antibody was evaluated by SDS-PAGE in reducing and non reducing conditions and compared with molecular weight standards (Fig. 3). The SDS-PAGE analysis in non reducing conditions indicates that monoclonal antibody 1G12 is constituted by an homogeneous band of 150,000 D as all immunoglobulins of IgG₁ class. This band is reduced by 2-mercaptoethanol in two bands of 50,000 and 25,000 D corresponding to the heavy and light chains of immunoglobulins.

Affinity: The approximate value of affinity constant of monoclonal antibody 1G12 for the fusion protein is 5 x 10⁹ M⁻¹ as evaluated by competitive enzyme immunoassay.

Method: 0.000125 mg of fusion protein in 50 »l/well was distributed onto 96-well microtiter plates and incubated overnight at 4°C. The day after the plates were washed with PBS pH 7.3 and then incubated with PBS-BSA 3% for 2 hours at room temperature (200 »l/well). The competition was made in solution between a fixed concentration of monoclonal antibody 1G12 corresponding to the 50% of the titration curve of the antibody and different concentrations of the fusion protein (from 0.1254 mg/ml to 0.00012 mg/ml) 1:1 volume and left to react 2 hours at 37°C. The product of the competition was then plated onto the microtiter wells and the amount of the 1G12 that did not react with the antigen in solution was titrated on the fusion protein with a sheep-anti-mouse F(ab')₂ antiserum conjugated with peroxidase following the protocol of the enzyme immunoassay used during the screening of monoclonal antibodies. The 50% value of the competition curve was assumed as an approximate value of the affinity constant of the antibody for the fusion protein (M.W. Steward, J. Steensgaard: "Antibody Affinity", CRC Press, Inc. Boca Raton, Florida, USA, 1983).

### Example 7: Use of the monoclonal antibody 1G12 in an enzyme immunoassay for detection of HIV-1 protease innibitory activity

In microtiter wells (for example "MICROELISA", from Greiner, FRG) 5 »l of samples which contain putative HIV-1 protease inhibitors were mixed with 40 »l of a mixture containing 0.25 M 2-(N-morpholino)ethane-sulfonic acid/sodium hydroxide buffer, pH 6.0, 0.6 % bovine serum albumin, 0.025 % Tween 20 and, preferentially, a cocktail of protease inhibitors to prevent non-specific proteolytic degradation by the proteases which might be present in the biological samples to be tested. As an example, this could be achieved adding 1-5 »g/ml leupeptin, 1-10 mM benzamidine, 0.5-1 mM sodium ethylene-diamino-tetraacetate (EDTA) and 0.2-1 mM phenyl-methyl-sulfonyl-fluoride (PMSF). All these protease inhibitors were found not to affect the activity of the HIV-1 protease at the indicated concentrations. 5 »l of conveniently diluted HIV-1 protease, or 5 »l of crude extract from recombinant E. coli expressing the HIV-1 protease were added to each well and the solutions were kept for a certain time (usually 5 to 20 minutes) at room temperature to allow binding of the putative inhibitors to the HIV-1 protease. 25 »l of conveniently diluted gal-gag 110 fusion protein of Example 2 were added to each well and the microtiter plates were incubated at 37°C for 40 minutes.
After incubation, 50 »l of solution from each well were transferred into the corresponding well of a similar microtiter plate on which monoclonal antibody 1G12 had previously been immobilized. Such binding of monoclonal antibody 1G12 to the plastic wells was achieved by incubating 50 »l of a 50-100 »g/ml solution of monoclonal antibody in each well of a similar microtiter plate (i.e. MICROELISA) for a time varying between 1 hour and overnight at room temperature. To block all the sites on the plastic which did not react with monoclonal antibody 1G12, 250 »l of a 3% solution of bovine serum albumin in PBS were added to each well and the microtiter plates were incubated at room temperature for 1 hour. After discarding all the solutions and washing 3-4 times with PBS, the plates were ready. The solutions containing the HIV-1 protease, the gal-gag 110 fusion protein and the putative inhibitors were incubated with the immobilized monoclonal antibody 1G12 for 2 hours at room temperature to allow the monoclonal antibody 1G12-mediated binding of the uncleaved fusion protein to the microtiter wells. The solutions were then discarded and the plates were washed 4-5 times with PBS containing 0.05% Tween 20. To detect the amount of β-galactosidase activity which remained bound to the wells, 240 »l of 1 mg/ml p-nitro-phenyl-β-D-galactopyranoside in 50 mM sodium chloride, 1 mM magnesium chloride and 70 mM 2-mercaptoethanol were added to each well. The microtiter plates were incubated at room temperature for 1 hour and then 60 »l of 1.5 M sodium carbonate were added to stop the β-galactosidase reaction. The absorbance at 405 nm was then measured spectrophotometrically using a microplate reader, for example the Titertek Multiscan model (Flow Laboratories, Irvine, Scotland, UK).
In such assay the control samples (no HIV-1 protease inhibitor) showed very low absorbance (background level) since all the gal-gag 110 fusion protein was cleaved by the HIV-1 protease and therefore was not bound by the immobilized antibody. On the contrary, when a HIV-1 protease innibitor was present the gal-gag 110 fusion protein was not cleaved and hence was bound by the immobilized antibody, yielding a much higher absorbance value.
In a representative experiment, this HIV-1 protease assay was checked with pepstatin (Sigma, St. Louis, Mo, USA), a known inhibitor of aspartic proteases like the HIV-1 protease. Absorbance values above background were obtained with as little as 1-2 »g/ml pepstatin, while at a concentration of 50-100 »g/ml the HIV-1 protease was completely inhibited.

### DESCRIPTION OF FIGURES

### Figure 1:

- (A): SDS-PAGE analysis of gal-gag 110 fusion protein before and after cleavage by HIV-1 protease.
- LANES:: MW St) molecular weight standards;
NO EX) purified gal-gag 110 fusion protein;
INC. TIME 0,15,30) gal-gag 110 fusion protein after incubation for 0, 15 and 30 minutes respectively at 37°C with E. coli crude extract containing HIV-1 protease;
NO PR) gal-gag 110 fusion protein after incubation for 30 minutes at 37°C with E. coli crude extract.

Gel electrophoresis was performed using a homogeneous 7.5% Phast-gel in a Phast-gel apparatus (Pharmacia, Uppsala, Sweden) following the manufacturer's instructions. 2-Mercaptoethanol was used as reducing agent. Coomassie Blue R-250 (BIORAD, Richmond, CA, USA) was used for staining.
- (B): Immuno-blot analysis of the gel in (A), using a monoclonal antibody anti-β-galactosidase (Promega, Madison, WI, USA), as primary antibody.
LANES order as in (A).

The transfer of proteins to nitrocellulose filter was performed by heating the gel at 70°C for 30 minutes, as suggested in the Phast-gel instruction manual. After blocking with 5% BSA (bovine serum albumin) in PBS for 1 hour at room temperature, the filter was incubated overnight with 1:1500 dilution of the anti-β-galactosidase antibody in PBS containing 0.25% BSA. The antibody bound to the filter was then detected by incubating with Gold-labelled Goat Anti-mouse serum (Janssen, Beerse, Belgium) followed by Silver Enhancement (Janssen).
- (C): Immuno-blot analysis of the gel in (A), using monoclonal antibody 1G12 as primary antibody.
LANES order as in (A).
Protein transfer and filter blocking were performed as in (B). The filter was then incubated overnight with 1:5 dilution of 1G12 cell supernatant in PBS containing 0.25% BSA. The detection of the bound antibody was performed as in (B).
- (D): Description of gal-gag 110 fusion protein

### Figure 2

Immuno-blot analysis of HIV-1 total viral proteins with human serum from an AIDS patient (lane a) or monoclonal antibody 1G12 (lane b).
Novapath Imuno-blot Strips (BIORAD, Richmond, CA, USA) were incubated for 1 hour at room temperature with (a) 1:100 dilution of human serum from an AIDS patient in PBS containing 0.25% BSA or (b) 1:4 dilution of 1G12 cell supernatant in PBS containing 0.25% BSA. The detection of bound antibodies was performed as in Figure 1B.

### Figure 3

It represents the SDS-PAGE analysis of monoclonal antibody 1G12 in non reducing (lanes 1, 2, 3) and reducing conditions (lanes 4, 5). Lanes : 1) molecular weight standards; 2) 1G12 ascitic fluid; 3) purified 1G12; 4) same as 3); 5) same as 1). Gel electrophoresis was performed using a 10-15% gradient Phast-gel in a Phast-gel apparatus, following the manufacturer's instructions. 2-Mercaptoethanol was used as reducing agent. Coomassie Blue R-250 was used for staining.

## Claims (Claims for the following Contracting State(s): AT, BE, CH, DE, DK, FR, GB, IT, LU, NL, SE, GR)

1. A monoclonal antibody against the HIV-1 gag protein precursor p55 characterized by its specificity toward a epitope containing the p24/p17 HIV-1 protease cleavage site.

2. The monoclonal antibody of claim 1, further characterized by the fact that:
a) its molecular weight is of the order of 150,000 Daltons
b) it belongs to the IgG₁ class and possesses light chains of the k type
c) it has an affinity constant of the approximate value of 5 x 10⁹ M⁻¹.

3. The antibody of any of claims 1 and 2, further characterized in that it has been produced using as antigen a HIV-gag fusion protein which contains 110 aminoacid residues fused with β-galactosidase and comprises a p55 fragment including the p24/p17 HIV-1 protease cleavage site (gal-gag 110 fusion protein).

4. A cell line producing the antibody of any of claims 1 to 3 which is a hybridoma.

5. The hybridoma of claim 4 which derives from fusion of a lymphoblastoid cell or a myeloma cell with an antibody producing cell from the spleen of animals immunized against the HIV-1 gag protein precursor p55 or a fragment thereof which contains the p24/p17 HIV-1 protease cleavage site.

6. The hybridoma of any of claims 4 and 5 wherein the immunized animals are small mammalians, preferably rats or mice and the fusion partners are syngenic.

7. The hybridoma of any of claims 4 to 6 wherein the myeloma cell line is HAT sensitive non-producer mouse myeloma cell line Sp 2/0-Ag14.

8. The hybridoma of any of claims 4 to 7 wherein the immunizing agent is a recombinant HIV-gag fusion protein containing a p55 fragment, which in turn comprises a portion of p24 and p17 and includes the p24/p17 HIV-1 protease cleavage site, bound to β-galactosidase.

9. The hybridoma of claim 8 wherein the immunizing agent is the gal-gag 110 fusion protein.

10. A process for preparing a monoclonal antibody of any of claims 1 to 3 which comprises:
(i) immunizing mice with a HIV-gag fusion protein containing the p55 sequence or a fragment thereof, which in turn comprises a portion of p24 and p17 and includes the p24/p17 HIV-1 protease cleavage site, bound to β-galactosidase; (ii) recovering spleen cells from immunized mice and fusing them with HAT sensitive non-producer mouse myeloma Sp 2/0-Ag 14 cells; (iii) testing the obtained hybrids by an appropriate immunoassay for the production of monoclonal antibodies against the p55 portion of the fusion protein; (iv) sub-cloning the cells positive to the previous assay; (v) analyzing the subcloned cells for the epitope specificity and selecting for larger cultivation those hybridomas which secrete an antibody directed specifically to the p24/p17 HIV-1 protease cleavage site of the fusion protein; (vi) isolating and purifying the specific antibody according to the methods usually employed in the art.

11. A process as in claim 10 wherein the immunizing agent is a fusion protein as in claim 10 which contains 110 aminoacid residues fused with β-galactosidase.

12. The antibody of any of claims 1 to 3 for use in an enzymatic immunoassay for the detection of the HIV-1 gag protein precursor p55 or a fragment thereof which contains the p24/p17 HIV-1 protease cleavage site, a natural or synthetic polypeptide containing the same aminoacid sequence of p55 or a fragment thereof which contains the p24/p17 HIV-1 protease cleavage site.

13. Use of the antibody of any of claims 1 to 3 for isolation and purification of the HIV-1 gag protein precursor p55 or a fragment thereof which contains the p24/p17 HIV-1 protease cleavage site, a natural or synthetic polypeptide containing the same aminoacid sequence of p55 or a fragment thereof which contains the p24/p17 HIV-1 protease cleavage site.

14. Use of the antibody of any of claims 1 to 3 in an affinity matrix for the isolation and purification according to claim 13.

15. The antibody according to any of claims 1 to 3 for use in screening HIV-1 protease inhibitory activity.

16. Use according to claim 15 wherein the antibody is employed in a sandwich ELISA-type assay.

17. The antibody of any of claims 1 to 3 for use in the diagnosis and monitoring of HIV-1 exposure.

18. Use of the monoclonal antibody of any of claims 1 to 3, according to claim 15 wherein the sample to be tested for HIV-protease inhibitory activity is selected from cellular extracts, microbial fermentation broths, solutions containing synthetic or natural products or extracts of biological material, including vegetals.

19. Use according to claim 18 wherein:
(a) the sample to be tested is incubated with a HIV-1 protease and HIV-gag fusion protein containing a p55 fragment, which in turn comprises a portion of both p24 and p17 and includes the p24/p17 HIV-1 protease cleavage site, bound to a marker enzyme, preferably β-galactosidase,
(b) the reaction mixture is incubated with an immobilized monoclonal antibody of any of claims 1 to 3,
(c) the resulting reaction mixture is separated from the immobilized antibody
(d) a reactant of the marker enzyme portion of the fusion protein, preferably a β-galactosidase-specific chromogenic substrate, is added to the immobilized monoclonal antibody
(e) the HIV-1 protease inhibitory activity is measured through the marker enzyme quantitation in comparison with a blank sample that does not contain the potential inhibitor.

20. Use according to claim 19 wherein the HIV-gag fusion protein is the gal-gag 110 fusion protein.

21. A kit for any of the uses of claims 12, 15, 16, 17, 18, 19 and 20, which contains the selective antibody of claims 1 to 3 associated with the other reagents and means necessary for performing analytical assay.

## Claims (Claims for the following Contracting State(s): ES)

1. A process for preparing a monoclonal antibody against the HIV-1 gag protein precursor p55 characterized by its specificity toward a epitope containing the p24/p17 HIV-1 protease cleavage site which comprises:
(i) immunizing mice with a HIV-gag fusion with protein containing the p55 sequence or a fragment thereof, which in turn comprises a portion of p24 and p17 and includes the p24/p17 HIV-1 protease cleavage site, bound to β-galactosidase; (ii) recovering spleen cells from immunized mice and fusing them with HAT sensitive non-producer mouse myeloma Sp 2/0-Ag 14 cells; (iii) testing the obtained hybrids by an appropriate immunoassay for the production of monoclonal antibodies against the p55 portion of the fusion protein; (iv) sub-cloning the cells positive to the previous assay; (v) analyzing the subcloned cells for the epitope specificity and selecting for larger cultivation those hybridomas which secrete an antibody directed specifically to the p24/p17 HIV-1 protease cleavage site of the fusion protein; (vi) isolating and purifying the specific antibody according to the methods usually employed in the art.

2. A process as in claim 1 wherein the immunizing agent is a fusion protein as in claim 1 which contains 110 aminoacid residues fused to β-galactosidase.

3. A process as in any of claims 1 and 2 further characterized in that the monoclonal antibody has the following characteristics:
a) its molecular weight is of the order of 150,000 Dalton
b) it belongs to the IgG₁ class and possesses light chains of the k type
c) it has an affinity constant of the approximate value of 5 x 10⁹ M⁻¹.

4. A process for screening HIV-1 protease inhibitory activity wherein:
(a) the sample to be tested is incubated with a HIV-1 protease and HIV-gag fusion protein containing a p55 fragment, which in turn comprises a portion of both p24 and p17 and includes the p24/p17 HIV-1 protease cleavage site, bound to a marker enzyme, preferably β-galactosidase,
(b) the reaction mixture is incubated with an immobilized monoclonal antibody produced according to any of claims 1 to 3,
(c) the resulting reaction mixture is separated from the immobilized antibody
(d) a reactant of the marker enzyme portion of the fusion protein, preferably a β-galactosidase-specific chromogenic substrate, is added to the immobilized monoclonal antibody
(e) the HIV-1 protease inhibitory activity is measured through the marker enzyme quantitation in comparison with a blank sample that does not contain the potential inhibitor.

## Patentansprüche (Patentansprüche für folgende(n) Vertragsstaat(en): AT, BE, CH, DE, DK, FR, GB, IL, LU, NL, SE, GR)

1. Monoclonaler Antikörper gegen die HIV-1-gag-Proteinvorstufe p55, gekennzeichnet durch seine Spezifität für ein Epitop, das die p24/p17-HIV-1-Proteasespaltstelle enthält.

2. Monoclonaler Antikörper nach Anspruch 1, weiter gekennzeichnet durch folgende Merkmale:
a) sein Molekulargewicht liegt in der Größenordnung von 150 000 Dalton;
b) er gehört zur IgG1-Klasse und besitzt leichte Ketten vom κ-Typ;
c) er weist eine Affinitätskonstante mit einem Wert von etwa 5 x 10⁹ M⁻¹ auf.

3. Antikörper nach Anspruch 1 oder 2, weiter dadurch gekennzeichnet, daß er unter Verwendung eines HIV-gag-Fusionsproteins als Antigen hergestellt wurde, das 110 mit β-Galactosidase fusionierte Aminosäurereste enthält und ein p55-Fragment umfaßt, das die p24/p17-HIV-1-Protease-spaltstelle enthält (gal-gag 110-Fusionsprotein).

4. Zellinie, die den Antikörper nach einem der Ansprüche 1 bis 3 produziert und ein Hybridom ist.

5. Hybridom nach Anspruch 4, das aus einer Fusion einer lyphopblastoiden Zelle oder einer Myelomzelle mit einer Antikörper-produzierenden Zelle aus der Milz von Tieren stammt, die gegen die HIV-1-gag-Proteinvorstufe p55 oder ein Fragment davon immunisiert wurden, die/das die p24/p17-HIV-1-Proteasespaltstelle enthält.

6. Hybridom nach Anspruch 4 oder 5, wobei die immunisierten Tiere kleine Säugetiere, vorzugsweise Ratten oder Mäuse, und die Fusionspartner syngen sind.

7. Hybridom nach einem der Ansprüche 4 bis 6, wobei die Myelomzellinie die HAT-sensitive, nicht-produzierende Mausmyelomzellinie Sp 2/0-Ag14 ist.

8. Hybridom nach einem der Ansprüche 4 bis 7, wobei das Immunogen ein rekombinantes HIV-gag-Fusionsprotein ist, das ein p55-Fragment enthält, das seinerseits einen Teil von p24 und p17 umfaßt und die p24/p17-HIV-1-Proteasespaltstelle enthält, gebunden an β-Galactosidase.

9. Hybridom nach Anspruch 8, wobei das Immunogen das gal-gag 110-Fusionsprotein ist.

10. Verfahren zur Herstellung eines monoclonalen Antikörpers nach einem der Ansprüche 1 bis 3, das die folgenden Schritte umfaßt:
(i) Immunisierung von Mäusen mit einem HIV-gag-Fusionsprotein, das die p55-Sequenz oder ein Fragment davon umfaßt, die/das ihrerseits/seinerseits einen Teil von p24 und p17 umfaßt und die p24/p17-HIV-1-Proteasespaltstelle enthält, gebunden an β-Galactosidase;
(ii) Entnahme von Milzzellen aus immunisierten Mäusen und deren Fusion mit HAT-sensitiven nicht-produzierenden Sp 2/0-Ag 14-Mausmyelomzellen;
(iii) Untersuchung der erhaltenen Hybride mittels eines geeigneten Immuntests auf die Produktion von monoclonalen Antikörpern gegen den p55-Anteil des Fusionsproteins;
(iv) Subclonierung der in dem vorherigen Test positiven Zellen;
(v) Analyse der subclonierten Zellen auf ihre Epitopspezifität und Auswahl derjenigen Hybridome für die Züchtung in größerem Umfang, die einen Antikörper sezernieren, der spezifisch gegen die p24/p17-HIV-1-Proteasespaltstelle des Fusionsproteins gerichtet ist;
(vi) Isolierung und Reinigung der spezifischen Antikörper nach Standardverfahren.

11. Verfahren nach Anspruch 10, wobei das Immunogen ein Fusionsprotein gemäß Anspruch 10 ist, das 110 Aminosäurereste, verknüpft mit β-Galactosidase, enthält.

12. Antikörper nach einem der Ansprüche 1 bis 3 zur Verwendung in einem enzymatischen Immuntest zum Nachweis der HIV-1-gag-Proteinvorstufe p55 oder eines Fragmentes davon, die/das die p24/p17-HIV-1-Protease-Spaltstelle enthält, eines natürlichen oder synthetischen Polypeptids, das dieselbe Aminosäuresequenz wie p55 enthält, oder eines Fragmentes davon, das die p24/p17-HIV-1-Proteasespaltstelle enthält.

13. Verwendung eines Antikörpers nach einem der Ansprüche 1 bis 3 zur Isolierung und Reinigung der HIV-1-gag-Proteinvorstufe p55 oder eines Fragmentes davon, die/das die p24/p17-HIV-1-Proteasespaltstelle enthält, eines natürlichen oder synthetischen Polypeptids, das dieselbe Aminosäuresequenz wie p55 enthält, oder eines Fragmentes davon, das die p24/p17-HIV-1-Proteasespaltstelle enthält.

14. Verwendung eines Antikörpers nach einem der Ansprüche 1 bis 3 in einer Affinitätsmatrix zur Isolierung und Reinigung nach Anspruch 13.

15. Antikörper nach einem der Ansprüche 1 bis 3 zur Verwendung beim Absuchen auf HIV-1-Proteaseinhibitoraktivität.

16. Verwendung nach Anspruch 15, wobei der Antikörper in einem Sandwich-ELISA-Test eingesetzt wird.

17. Antikörper nach einem der Ansprüche 1 bis 3 zur Verwendung in der Diagnose und Überwachung von HIV-1-Exponierung.

18. Verwendung des monoclonalen Antikörpers nach einem der Ansprüche 1 bis 3 gemäß Anspruch 15, wobei die auf HIV-Proteaseinhibitoraktivität zu testende Probe ausgewählt ist aus Zellextrakten, mikrobiellen Fermentationsbrühen, synthetische oder natürliche Produkte enthaltenden Lösungen oder Extrakten aus biologischen Materialien, einschließlich Pflanzen.

19. Verwendung nach Anspruch 18, wobei
(a) die zu testende Probe inkubiert wird mit einer HIV-1-Protease und einem HIV-gag-Fusionsprotein, das ein p55-Fragment enthält, das seinerseits einen Teil von p24 und p17 umfaßt und die p24/p17-Proteasespaltstelle enthält, gebunden an ein Markerenzym, vorzugsweise β-Galactosidase;
(b) das Reaktionsgemisch mit einem immobilisierten monoclonalen Antikörper nach einem der Ansprüche 1 bis 3 inkubiert wird;
(c) das so erhaltene Reaktionsgemisch vom immobilisierten Antikörper abgetrennt wird;
(d) ein Reaktionspartner des Markerenzymanteils des Fusionsproteins, vorzugsweise ein chromogenes Substrat spezifisch für β-Galactosidase, zu dem immobilisierten monoclonalen Antikörper zugesetzt wird; und
(e) die HIV-1-Proteaseinhibitoraktivität durch Quantifizierung des Markerenzyms gemessen wird, wobei als Vergleich eine Leerprobe herangezogen wird, die den potentiellen Inhibitor nicht enthält.

20. Verwendung nach Anspruch 19, wobei das HIV-gag-Fusionsprotein das gal-gag 110-Fusionsprotein ist.

21. Kit für die Verwendung nach einem der Ansprüche 12, 15, 16, 17, 18, 19 und 20, der den selektiven Antikörper nach einem der Ansprüche 1 bis 3 in Verbindung mit den anderen Reagentien und Mitteln enthält, die für die Durchführung eines analytischen Tests notwendig sind.

## Patentansprüche (Patentansprüche für folgende(n) Vertragsstaat(en): ES)

1. Verfahren zur Herstellung eines monoclonalen Antikörpers gegen die HIV-1-gag-Proteinvorstufe p55, der durch seine Spezifität für ein Epitop gekennzeichnet ist, welches die p24/p17-HIV-1-Proteasespaltstelle enthält, das umfaßt:
(i) Immunisierung von Mäusen mit einem HIV-gag-Fusionsprotein, das die p55-Sequenz oder ein Fragment davon umfaßt, die/das ihrerseits/seinerseits einen Teil von p24 und p17 umfaßt und die p24/p17-HIV-1-Proteasespaltstelle enthält, gebunden an β-Galactosidase;
(ii) Entnahme von Milzzellen aus immunisierten Mäusen und deren Fusion mit HAT-sensitiven nicht-produzierenden Sp 2/0-Ag 14-Mausmyelomzellen;
(iii) Untersuchung der erhaltenen Hybride mittels eines geeigneten Immuntests auf die Produktion von monoclonalen Antikörpern gegen den p55-Anteil des Fusionsproteins;
(iv) Subclonierung der in dem vorherigen Test positiven Zellen;
(v) Analyse der subclonierten Zellen auf ihre Epitopspezifität und Auswahl derjenigen Hybridome für die Züchtung in größerem Umfang, die einen Antikörper sezernieren, der spezifisch gegen die p24/p17-HIV-1-Proteasespaltstelle des Fusionsproteins gerichtet ist;
(vi) Isolierung und Reinigung der spezifischen Antikörper nach Standardverfahren.

2. Verfahren nach Anspruch 1, wobei das Immunogen ein Fusionsprotein gemäß Anspruch 1 ist, das 110 Aminosäurereste, verknüpft mit β-Galactosidase, enthält.

3. Verfahren nach Anspruch 1 oder 2, ferner dadurch gekennzeichnet, daß der monoclonale Antikörper die folgenden Merkmale aufweist:
a) sein Molekulargewicht liegt in der Größenordnung von 150 000 Dalton;
b) er gehört zur IgG1-Klasse und besitzt leichte Ketten vom κ-Typ;
c) er weist eine Affinitätskonstante mit einem Wert von etwa 5 x 10⁹ M⁻¹ auf.

4. Verfahren zum Absuchen auf HIV-1-Proteaseinhibitoraktivität, wobei
(a) die zu testende Probe inkubiert wird mit einer HIV-1-Protease und einem HIV-gag-Fusionsprotein, das ein p55-Fragment enthält, das seinerseits einen Teil von p24 und p17 umfaßt und die p24/p17-Proteasespaltstelle enthält, gebunden an ein Markerenzym, vorzugsweise β-Galactosidase;
(b) das Reaktionsgemisch mit einem immobilisierten monoclonalen Antikörper nach einem der Ansprüche 1 bis 3 inkubiert wird;
(c) das so erhaltene Reaktionsgemisch vom immobilisierten Antikörper abgetrennt wird;
(d) ein Reaktionspartner des Markerenzymanteils des Fusionsproteins, vorzugsweise ein chromogenes Substrat spezifisch für β-Galactosidase, zu dem immobilisierten monoclonalen Antikörper zugesetzt wird; und
(e) die HIV-1-Proteaseinhibitoraktivität durch Quantifizierung des Markerenzyms gemessen wird, wobei als Vergleich eine Leerprobe herangezogen wird, die den potentiellen Inhibitor nicht enthält.

## Revendications (Revendications pour l'(les) Etat(s) contractant(s) suivant(s): AT, BE, CH, DE, DK, FR, GB, IT, LU, NL, SE, GR)

1. Anticorps monoclonal dirigé contre le précurseur p55 de la protéine gag de HIV-1, caractérisé par sa spécificité à l'égard d'un épitope contenant le site de coupure p24/p17 par la protéase de HIV-1.

2. Anticorps monoclonal selon la revendication 1, caractérisé en outre par le fait que:
a) sa masse moléculaire est de l'ordre de 150 000 daltons,
b) il appartient à la classe IgG₁ et possède des chaînes légères du type κ,
c) il a une constante d'affinité d'environ 5×10⁹ M⁻¹.

3. Anticorps selon l'une quelconque des revendications 1 et 2, caractérisé en outre en ce qu'il a été produit avec utilisation, en tant qu'antigène, d'une protéine de fusion 110 HIV-gag qui contient 110 restes aminoacide fusionnés avec la β-galactosidase, et comprend un fragment p55 comprenant le site de coupure p24/p17 par la protéase de HIV-1 (protéine de fusion gal-gag 110).

4. Lignée cellulaire produisant l'anticorps selon l'une quelconque des revendications 1 à 3, qui est une lignée d'hybridomes.

5. Hybridome de la revendication 4, qui provient de la fusion d'une cellule lymphoblastoïde ou d'une cellule de myélome avec une cellule productrice d'anticorps provenant de la rate d'animaux immunisés contre le précurseur p55 de la protéine gag de HIV-1 ou un fragment de celui-ci qui contient le site de coupure p24/p17 par la protéase de HIV-1.

6. Hybridome selon l'une quelconque des revendications 4 et 5, dans lequel des animaux immunisés sont de petits mammifères, de préférence des rats ou des souris, et les partenaires de fusion sont syngéniques.

7. Hybridome selon l'une quelconque des revendications 4 à 6, dans lequel la lignée cellulaire de myélome est la lignée cellulaire Sp2/0-Ag14 de myélome murin non productrice et sensible à HAT.

8. Hybridome selon l'une quelconque des revendications 4 à 7, dans lequel l'agent immunisant est une protéine de fusion HIV-gag recombinante contenant un fragment p55, qui à son tour comprend une partie de p24/p17 et comprend le site de coupure p24/p17 par la protéase de HIV-1, lié à la β-galactosidase.

9. Hybridome selon la revendication 8, dans lequel l'agent immunisant est la protéine de fusion gal-gag 110.

10. Procédé pour la production d'un anticorps monoclonal selon l'une quelconque des revendications 1 à 3, comprenant: (I) l'immunisation de souris avec une protéine de fusion HIV-gag contenant la séquence de p55 ou un fragment de celle-ci, qui à son tour comprend une partie de p24 et p17 et comprend le site de coupure p24/p17 par la protéase de HIV-1, lié à la β-galactosidase; (II) la récupération de cellules spléniques à partir de souris immunisées et leur fusion avec des cellules Sp2/0-Ag14 de myélome murin non productrices et sensibles à HAT; (III) l'essai des hybrides obtenus, à l'aide d'un essai immunologique approprié, pour la mise en évidence de la production d'anticorps monoclonaux dirigés contre la portion p55 de la protéine de fusion; (IV) le sous-clonage des cellules positives dans l'essai précédent; (V) l'analyse des cellules sous-clonées pour la détermination de la spécificité à l'égard de l'épitope, et la sélection pour une culture à plus grande échelle de ces hybridomes qui sécrètent un anticorps dirigé spécifiquement contre le site de coupure p24/p17 par la protéase de HIV-1 de la protéine de fusion; (VI) l'izolement et la purlfication de l' anticorps spécifique, selon les méthodes habituellement utilisées dans la technique.

11. Procédé selon la revendication 10, dans lequel l'agent immunisant est une protéine de fusion telle que dans la revendication 10, qui contient 110 restes aminoacide fusionnés avec la β-galactosidase.

12. Anticorps selon l'une quelconque des revendications 1 à 3, pour utilisation dans un essai immunoenzymatique pour la détection du précurseur p55 de la protéine gag de HIV-1 ou d'un fragment de celui-ci qui contient le site de coupure p24/p17 par la protéase de HIV-1, d'un polypeptide naturel ou synthétique contenant la même séquence d' aminoacides que p55 ou d'un fragment de celui-ci qui contient le site de coupure p24/p17 par la protéase de HIV-1.

13. Utilisation de l'anticorps selon l'une quelconque des revendications 1 à 3, pour l'isolement et la purification du précurseur p55 de la protéine gag de HIV-1 ou d'un fragment de celui-ci qui contient le site de coupure p24/p17 par la protéase de HIV-1, d'un polypeptide naturel ou synthétique contenant la même séquence d'aminoacides que p55 ou d'un fragment de celui-ci qui contient le site de coupure p24/p17 par la protéase de HIV-1.

14. Utilisation de l'anticorps selon l'une quelconque des revendications 1 à 3, dans une matrice d'affinité pour l'isolement et la purification selon la revendication 13.

15. Anticorps selon l'une quelconque des revendications 1 à 3, pour utilisation dans l'essai de l'activité inhibitrice de protéase de HIV-1.

16. Utilisation selon la revendication 15, dans laquelle l'anticorps est employé dans un essai de type ELISA en sandwich.

17. Anticorps selon l'une quelconque des revendications 1 à 3, pour utilisation dans le diagnostic et le contrôle de l'exposition au HIV-1.

18. Utilisation de l'anticorps monoclonal de l'une quelconque des revendications 1 à 3, selon la revendication 15, dans laquelle l'échantillon à tester pour détecter l'activité inhibitrice de protéase de HIV est choisi parmi des extraits cellulaires, des bouillons de fermentation microbiens, des solutions contenant des produits naturels ou synthétiques, ou des extraits de matériels biologiques, compris de végétaux.

19. Utilisation selon la revendication 18, dans laquelle:
(a) l'échantillon à tester est mis à incuber avec une protéase de HIV-1 et une protéine de fusion HIV-gag contenant un fragment p55, qui a son tour comprend une portion à la fois de p24 et de p17 et comprend le site de coupure p24/p17 par la protéase de HIV-1, lié à une enzyme de marquage, de préférence la β-galactosidase,
(b) on met le mélange réactionnel à incuber avec un anticorps monoclonal immobilisé selon l'une quelconque des revendications 1 à 3,
(c) on sépare le mélange réactionnel résultant d'avec l'anticorps immobilisé,
(d) on ajoute à l'anticorps monoclonal immobilisé un réactif de la portion enzyme de marquage de la protéine de fusion, de préférence un substrat chromogène spécifique pour la β-galatosidase,
(e) on mesure l'activité inhibitrice de protéase de HIV-1, ou moyen de la détermination quantitative de l' enzyme de marquage, par comparaison avec un échantillon blanc qui ne contient pas l'inhibiteur potentiel.

20. Utilisation selon la revendication 19, dans laquelle la protéine de fusion HIV-gag est la protéine de fusion gal-gag 110.

21. Nécessaire pour l'une quelconque des utilisations des revendications 12, 15, 16, 17, 18, 19 et 20, qui contient l'anticorps sélectif des revendications 1 à 3, associé aux autres réactifs et moyens nécessaires pour effectuer un essai analytique.

## Revendications (Revendications pour l'(les) Etat(s) contractant(s) suivant(s): ES)

1. Procédé pour la production d'un anticorps monoclonal dirigé contre le précurseur p55 de la protéine gag de HIV-1, caractérisé par sa spécificité pour un épitope contenant le site de coupure p24/p17 par la protéase de HIV-1, comprenant: (I) l'immunisation de souris avec une protéine de fusion HIV-gag contenant la séquence de p55 ou un fragment de celle-ci, qui à son tour comprend une partie de p24/p17 et comprend le site de coupure p24/p17 par la protéase de HIV-1, lié à la β-galactosidase; (II) la récupération de cellules spléniques à partir de souris immunisées et leur fusion avec des cellules Sp2/0-Ag14 de myélome murin non productrices et sensibles à HAT; (III) l'essai des hybrides obtenus, à l'aide d'un essai immunologique approprié, pour la mise en évidence de la production d'anticorps monoclonaux dirigés contre la portion p55 de la protéine de fusion; (IV) le sous-clonage des cellules positives dans l'essai précédent; (V) l'analyse des cellules sous-clonées pour la détermination de la spécificité à l'égard de l'épitope, et la sélection pour une culture à plus grande échelle de ces hybridomes qui sécrètent un anticorps dirigé spécifiquement contre le site de coupure p24/p17 par la protéase de HIV-1 de la protéine de fusion; (VI) l'isolement et la purification de l'anticorps spécifique, selon les méthodes habituellement utilisées dans la technique.

2. Procédé selon la revendication 1, dans lequel l'agent immunisant est une protéine de fusion telle que dans la revendication 1, qui contient 110 restes aminoacide fusionnés avec la β-galactosidase.

3. Procédé selon l'une quelconque des revendications 1 et 2, caractérisé en outre en ce que l'anticorps monoclonal a les caractéristiques suivantes:
a) sa masse moléculaire est de l'ordre de 150 000 daltons,
b) il appartient à la classe IgG₁ et possède des chaînes légères du type κ,
c) il a une constante d'affinité d'environ 5×10⁹ M⁻¹.

4. Procédé pour l'essai d'une activité inhibitrice à l'égard de la protéase HIV-1, dans lequel:
(a) l'échantillon à tester est mis à incuber avec une protéase de HIV-1 et une protéine de fusion HIV-gag contenant un fragment p55, qui a son tour comprend une portion à la fois de p24 et de p17 et comprend le site de coupure p24/p17 par la protéase de HIV-1, lié à une enzyme de marquage, de préférence la β-galactosidase,
(b) on met le mélange réactionnel à incuber avec un anticorps monoclonal immobilisé selon l'une quelconque des revendications 1 à 3,
(c) on sépare l'échantillon résultant d'avec l'anticorps immobilisé,
(d) on ajoute à l'anticorps monoclonal immobilisé un réactif de la portion enzyme de marquage de la protéine de fusion, de préférence un substrat chromogène spécifique pour la β-galactosidase,
(e) on mesure l'activité inhibitrice de protéase de HIV-1, ou moyen de la détermination quantitative de l'enzyme de marquage, par comparaison avec un échantillon blanc qui ne contient pas l'inhibiteur potentiel.
